# EUROPEAN PATENT SPECIFICATION

(11) **EP 3 326 518 B1**
(45) Date of publication and mention of the grant of the patent: **11.03.2020**
(21) Application number: 17020544.7
(22) Date of filing: 21.11.2017
(51) Int. Cl.: A61B 5/00, A61B 5/11, G01C 3/00, A61B 5/107

(54) **WEARABLE DEVICE FOR MONITORING BODY POSTURE**
TRAGBARE VORRICHTUNG ZUR ÜBERWACHUNG DER KÖRPERHALTUNG
DISPOSITIF PORTABLE PERMETTANT DE SURVEILLER LA POSTURE CORPORELLE

(30) Priority: 28.11.2016 SE 1630276
(43) Date of publication of application: 30.05.2018
(73) Proprietor: Fixaposture AB (559045-5704), 223 70 Lund (SE)
(72) Inventor: Nybom, Lars Göran, 247 35 Södra Sandby (SE); Nybom, Nils Viktor, 222 22 Lund (SE); Harrie, Jonas Per, 429 31 Kullavik (SE)

(56) References cited:
- WO-A1-2015/007132
- WO-A2-2017/137852
- US-A1- 2009 054 814
- US-B2- 8 157 752
- Jeon Sanghoon ET AL: "Prevention System and Forward Head Posture Using IMU and Infrared Distance Sensor", , 14 November 2015 (2015-11-14), pages 449-452, XP055456619, Retrieved from the Internet: URL:http://cps.dgist.ac.kr/wp-content/uplo ads/2015/09/2015_C_28.pdf [retrieved on 2018-03-06]
- Inukai Kazuma ET AL: "Estimating head posture from point cloud data of lower chin", Transactions of the Virtual Reality Society of Japan, 1 February 2017 (2017-02-01), pages 605-612, XP055456284, DOI: https://doi.org/10.18974/tvrsj.21.4_605 Retrieved from the Internet: URL:https://www.jstage.jst.go.jp/article/t vrsj/21/4/21_605/_article/-char/en [retrieved on 2018-03-05]
- INUKAI KAZUMA ET AL: "Estimate head posture with neck dangling device", ASIA PACIFIC WORKSHOP ON MIXED REALITY 2016 (APMR 2016), 24 April 2016 (2016-04-24), XP055572350, Andong, Korea

## Description

### TECHNICAL FIELD

The present invention relates to a wearable device for monitoring body posture comprising a housing exhibiting a back surface and a front surface, a fastening means adapted to secure the housing to a user's body, a first sensor device and a second sensor device each being mounted at the wearable device and adapted to measure changes in a user's body posture when the wearable device is worn by the user, a processing unit adapted to collect and process sensor signals delivered from said sensor devices and to generate and transmit output signals, and at least one feedback device adapted to receive said output signals from the processing unit and to provide posture feedback to the user.

### BACKGROUND OF THE INVENTION

The importance of physical activity and proper posture for physical appearance and health has long been appreciated. It is known that it is important to maintain a proper posture of the cervical, thoracic and lumbar regions of the spine when standing or sitting for long periods of time. It is also known that maintaining a proper posture can be vital for a successful rehabilitation of herniated disc patients and patients with whiplash injury.

Still, many people spend long hours sitting or standing still in unhealthy postures, both at work and during leisure time. It has been found that users of computers, hand-held electronic devices and bifocals, as well as people doing a lot of reading, may develop posture related disorders such as vision problems and musculoskeletal problems. So-called forward head posture places a lot of stress on the cervical spine, especially the discs, and can have a serious negative impact on the health and wellbeing of an affected person. Maintaining the cervical spine in a neutral position is fundamental to maintaining proper posture of the thoracic and lumbar spine and preventing serious musculoskeletal disorders and disc herniations. Developing forward head posture will therefore have a negative effect on the whole spine and can also affect other organs of the body.

A number of posture monitoring and correcting systems and devices intended to reduce the problems caused by poor posture have been proposed previously.

For example, the international patent application WO2016/079585 A1 discloses a wearable posture monitoring and feedback system, and a related method, configured to monitor and provide feedback regarding a user's posture while operating and viewing a portable electronic device. The disclosed system includes a wearable frame configured to be fit about a head of the user, a sensor mounted on the wearable frame configured to monitor at least a head position of the user, and a transmitter configured to transmit data related to at least the head position wirelessly from the sensor to a receiver on or in the portable electronic device. The system is configured to provide feedback to the user based on the data from the sensor and send a signal to alter an output provided to the user by the portable electronic device based on the data from the sensor.

Furthermore, US Patent No. 5,469,861 discloses a method and an apparatus for monitoring posture where a first unit is attached to a first position on a user's body, and a second unit is attached to a second position on the user's body. A signal such as an ultrasonic signal is transmitted by the first unit to the second unit. In response, the second unit transmits a signal back to the first unit. The round-trip travel time of the signals is proportional to the distance between the units. If the distance differs from an ideal distance by more than a maximum allowable amount, improper posture is indicated to the user by an alarm signal.

Still further, US Patent No. 5,425,378 discloses a monitoring unit mounted atop headgear fitted with a proportional level sensor which detects head inclination in the sagittal plane. A battery powered microcontroller, interfaced with the level sensor, monitors the head tilt and provides a sonic indication whenever the tilt exceeds pre-set limits to the front or rear. The use of a proportional level sensor of the capacitive/fluid type enables the microcontroller to be configured to allow the user to preset different tilt limits, and to select different modes, for example beginning or advanced training, sitting, standing or exercising body positions, and to set up a designated training session duration, timed by sonic indication, during which the number and direction of head tilt deviations detected may be counted. Such statistical data may be displayed to the user on a LCD display. With the level sensor mounted directly to one side of the headgear, the monitor unit in a housing atop the headgear may specially be non-rigidly attached to provide a degree of wobble or totter perceptible to the user as kinaesthetic/proprioceptive feedback, in addition to the audible biological feedback received from the sonic tilt limit indication. The microcontroller system may be specially configured to recognize and ignore gross movements such as reaching and bending, while still feeding back to the user the finer postural deviations.

Furthermore, an article with the title "Prevention System and Forward Head Posture Using IMU and Infrared Distance Sensor" by Jeon Sanghoon et al., 14 November 2015, pages 449-452, retrieved from the Internet: URL:http://cps.dgist.ac.kr/wp-content/uploads/2015/09/2015_C_28.pdf discloses a wearable device to put behind a user's ear comprising three sensors such as IMU, IR distance and vibration sensors. The IMU sensor is used to detect sensor positions and the IR distance sensor is used to measure the vertical distance between the tragus of the ear and the torso of the user. The vibration sensor gives a vibration warning when the system detects Forward Head Posture.

The article "Estimating head posture with neck dangling device" by Kazuma Inukai and Nobuchika Sakata, 24 April 2016, ASIA PACIFIC WORKSHOP ON MIXED REALITY 2016 (APMR 2016), Andong, Korea, discloses the estimation of head posture using a depth sensor integrated into a necklace type device.

In addition, WO 2017/137852 A2, published on 17 August 2017, discloses a posture monitoring apparatus comprising at least one processor, and at least one sensor configured to sense position an posture of a wearer of the device. The at least one processor receives and processes input from the at least one sensor, and when necessary, indicates the wearer's posture needs correction, and operates and alarm module to signal the wearer to correct the wearer's posture, wherein the apparatus is worn above the shoulders of the wearer.

Many of the previously known posture monitoring devices try to estimate a user's posture by measuring the inclination, or tilt angle, of a user's head or cervical spine region. A drawback of this approach is that such measurements are not sufficient to quantify cervical spine posture and may fail to detect a forward translation of the head of the user. This means that a user of such a posture monitoring device runs the risk of developing a forward head posture without receiving any warning or feedback thereof from the device.

Another drawback of many of the previously known posture monitoring devices is that they do not provide any definite posture values, which makes monitoring and documentation of a user's training progress difficult or impossible.

Still another drawback of many of the previously known posture monitoring devices is that they usually consist of several separate units which can be difficult to put on, connect and use for the user and have a design which makes them difficult to adjust to different body sizes and uncomfortable to wear for the user. Furthermore, many of the previously known devices are expensive, bulky and more conspicuous to other people than necessary.

### SUMMARY OF THE INVENTION

A first object of the present invention is therefore to provide a small, simple, inconspicuous and affordable wearable device for monitoring body posture which enables precise monitoring of neck and upper back posture and provision of feedback thereof to the user, and which ensures reliable detection of forward translation of the head so that the user can be alerted to this condition.

This first object is achieved by means of a wearable device for monitoring body posture according to claim 1, which comprises a housing exhibiting a back surface and a front surface, a fastening means adapted to secure the housing to a user's body, a first sensor device and a second sensor device each being mounted at the wearable device and adapted to measure changes in a user's body posture when the wearable device is worn by a user, a processing unit adapted to collect and process sensor signals delivered from said sensor devices and to generate and transmit output signals, and at least one feedback device adapted to receive said output signals from the processing unit and to provide posture feedback to a user wearing the wearable device, wherein the processing unit is configured to decide when to transmit a control signal to the feedback device causing it to provide posture feedback to a user, based upon said signals delivered by the sensors, and wherein the at least one feedback device is configured to provide posture feedback when receiving the control signal from the processing unit, wherein the fastening means is adapted to secure the housing adjacent to a user's sternum with the back surface facing a user's body when the wearable device is worn, wherein the first sensor device is mounted at the housing and configured to deliver a signal corresponding to an actual distance between the front surface of the housing and a user's chin when the wearable device is worn, wherein the second sensor device is mounted at the housing and configured to deliver a signal corresponding to an actual inclination of a user's sternum when the wearable device is worn, and that the processing unit is configured to, when the wearable device is worn by a user, determine both inclination and translation of a user's head by calculating both head tilt and displacement based on the signals from the second sensor device corresponding to the inclination of the sternum, and the signals from the first sensor device corresponding to the distance between the front surface of the housing and the chin.

By providing the wearable device for monitoring body posture with a fastening means adapted to secure the housing adjacent to the user's sternum with the back surface facing the user's body, a first sensor device mounted at the housing and configured to deliver a signal corresponding to an actual distance between the front surface of the housing and the user's chin, and a second sensor device mounted at the housing and configured to deliver a signal corresponding to an actual inclination of the user's sternum, the wearable device can detect the actual distance between the housing and the user's chin and the actual inclination of the user's sternum simultaneously and therefrom calculate accurate and definite values for both inclination and displacement of the user's head. Based on the actual inclination of the user's sternum and the calculated tilt and displacement of the user's head, the wearable device can reliably quantify cervical spine posture and also detect forward translation of the head, and decide when to cause the feedback device to provide posture feedback to the user. Such a design of the wearable device eliminates the risk of the user assuming a forward head posture without receiving any warning or feedback thereof from the device.

Further objects and advantages of the invention, and the features enabling these objects and advantages to be achieved, will become evident from the following description. The invention is defined by an apparatus according to independent claim 1.

### BRIEF DESCRIPTION OF THE DRAWINGS

In the following, the present invention will be described by means of a number of different embodiments, with reference to the accompanying drawings, in which:
Figure 1 is a schematic illustration showing how a wearable device for monitoring body posture according to the invention is worn by a user;
Figure 2A shows a perspective view of a wearable device according to a preferred embodiment of the invention, with the fastening means removed;
Figure 2B shows a different perspective view of the wearable device in Figure 2A; and
Figures 3A-3G schematically illustrate different head, neck and spine postures of a person, which can be monitored and if necessary corrected by means of the wearable device according to the invention, wherein Figure 3D shows the person sitting with good posture.

### DESCRIPTION OF EMBODIMENTS OF THE INVENTION

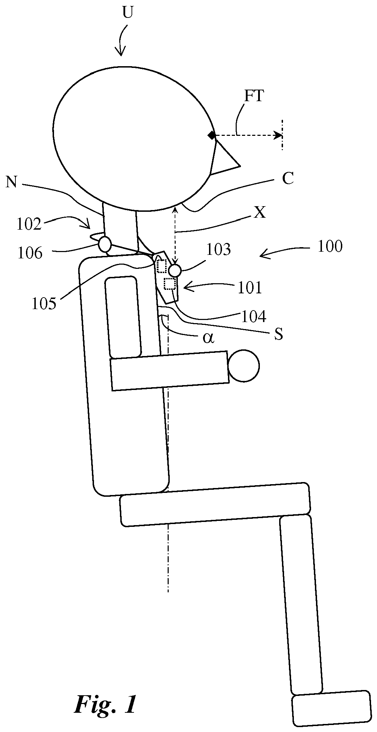

In the following, a number of embodiments of a device according to the invention will be described in greater detail with reference to the accompanying Figures 1-3G.

Figure 1 schematically illustrates a wearable device 100 for monitoring body posture according to the invention, when worn by a user U.

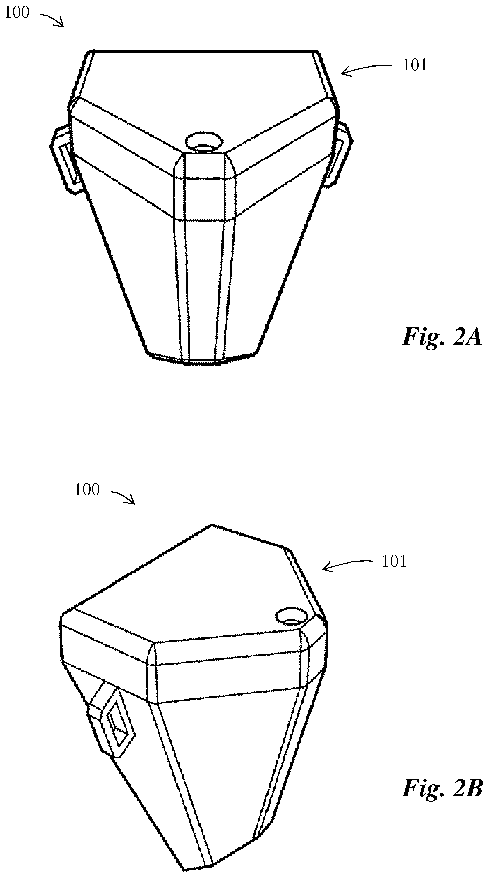

The wearable device 100 according to the invention comprises a housing 101 (also illustrated in Figs. 2A-2B) exhibiting a back surface and a front surface, and a fastening means 102 (illustrated in Fig. 1) adapted to secure the housing 101 to a user's body.

The wearable device 100 further comprises a first sensor device 103 and a second sensor device 104 each being mounted at the wearable device and adapted to measure changes in a user's body posture when the wearable device 100 is worn by the user U, and a processing unit adapted to collect and process sensor signals delivered from said sensor devices 103, 104 and to generate and transmit output signals. The wearable device 100 also comprises at least one feedback device adapted to receive said output signals from the processing unit and to provide posture feedback to the user.

The fastening means 102 of the wearable device 100 according to the invention is adapted to secure the housing 101 adjacent to a user's sternum S with the back surface facing the user's body. The fastening means can particularly advantageously comprise a carrying strap 102 adapted to be fitted around a user's neck N, wherein the carrying strap preferably comprises one or several fastening elements (not shown in the drawings) adapted to allow adjustment and locking of strap length. The provision of a carrying strap with one or several such fastening elements, for example strap buckles or pairs of magnets, facilitates adjustment to body size and repeated use of the wearable device for the user, and allows the user to inconspicuously wear the inexpensively designed and small-dimensioned housing of the device suspended on his/her chest from his/her neck. The carrying strap can be semi rigid, i e made of a gooseneck tube, in a part of its length or of the whole length, to be able to accommodate the curvature of the neck and upper back region, to thereby obtain a more stable support. However, within the scope of the present invention, it is also conceivable with other fastening means than a neck carrying strap, for example fastening means comprising suitably designed clips or clamps, adhesive tape for attachment to a user's clothing or skin, hook and loop tape, a belt for attachment around the user's body, or magnets acting through the user's clothing.

The first sensor device 103 of the wearable device 100 according to the invention is mounted at the housing 101 and configured to deliver a signal related to an actual distance X between the front surface of the housing 101 and a user's chin C, while the second sensor device 104 is mounted at the housing 101 and configured to deliver a signal related to an actual inclination α of the user's sternum S.

The processing unit 105 of the wearable device 100 according to the invention is configured to decide when to transmit a control signal to the feedback device 106 causing it to provide posture feedback to the user U, based upon said signals delivered by the sensors.

The processing unit 105 is configured to determine both inclination and translation FT of a user's head based upon said signals delivered by the sensors. The processing unit calculates both tilt and displacement of the user's head based on the signals from the second sensor device 104, corresponding to the inclination of the user's sternum S, and the signals from the first sensor device 103, corresponding to the distance between the housing 101 positioned at the user's sternum S and the user's chin C.

The at least one feedback device 106 of the wearable device 100 according to the invention is configured to provide posture feedback to the user U when receiving the control signal from the processing unit 105. The feedback device 106 preferably comprises a vibrating element intended to be in contact with the user's body when the wearable device 100 is worn by the user U. Alternatively or optionally, the feedback device can comprise a loudspeaker for audible feedback and/or a LED light device for visual feedback.

Advantageously, the carrying strap 102 has the feedback device 106 adjustably mounted thereon to allow the feedback device to be placed in a position that is close to the middle of the back of the user's neck N when the wearable device 100 is worn by the user U. Such an embodiment is particularly advantageous since the feedback can be provided directly to the muscles which have to change their muscle memory. However, it also conceivable with embodiments of the invention where the feedback device is instead disposed in or at the housing of the wearable device.

In another advantageous embodiment of the invention, the housing 101 exhibits sloping side surfaces connecting said back surface to said front surface, so that the back surface and the side surfaces together form a wedge-shape adapted to increase friction against a user's clothing. Such a design helps to keep the wearable device 100 in an intended defined position on the user's chest.

In a preferred embodiment of the invention, the first sensor device is a contactless distance measurement sensor 103 mounted at the front surface of the housing 101. A contactless distance measurement sensor mounted in such a position enables an accurate measurement of the actual distance X between the front surface of the housing 101 and the user's chin C. The contactless distance measurement sensor is particularly advantageously an infrared sensor or a radar sensor, preferably a time-of-flight sensor.

In a preferred embodiment of the invention, the second sensor device is an inclination measurement sensor 104 mounted in the housing 101. An inclination measurement sensor mounted in the housing enables an accurate measurement of the actual inclination α of the user's sternum S. The inclination measurement sensor is advantageously an inclinometer or a tilt sensor.

In another advantageous embodiment, the wearable device 100 further comprises a third sensor device (not shown in the figures) in the form of an accelerometer configured to measure a user's motion activity. The accelerometer can alternatively or additionally provide other information, such as inclination data.

In one advantageous embodiment of the invention, the wearable device 100 is provided with an ON/OFF power switch in the form of an electrical connector with mating separable contacts, a mechanical device actuating a switch in the wearable device, or a magnet-actuated switch, which ON/OFF power switch is mounted on or integrated with the fastening means 102. Such a design allows the wearable device 100 to be switched on automatically, for example, when the user puts a carrying strap 102 of the wearable device around his/her neck, and to be switched off automatically when the user removes the wearable device.

In a particularly advantageous embodiment of the invention, the wearable device 100 is provided with a computer system, which may include said processing unit 105, wherein the computer system further comprises a non-volatile memory for storing data on a user's training development, a radio communication unit for transmitting data to and receiving data from an external unit, and an interface for controlling a user interface of the wearable device 100. In such an embodiment, the computer system advantageously can be used for analyzing sensor signals and other data, to decide when to send feedback to the user, and to adjust alarm sensitivity for motion activity. The external unit can advantageously be used for special uses, including for setting time delays and alarm limits, for visual presentation of actual status and historical data, for specialist supervision, for definition of a rehabilitation program, for providing a reminder when it is time for the next training session, for initiating an alarm signal and for storing historical data and events.

Preferably, the wearable device 100 has an integrated user interface, which may include said vibrating element 106, said loudspeaker for audible feedback, said LED light device for visual feedback and/or said ON/OFF power switch, and which further comprises LED devices for indication of operational state, battery charging state and/or ON/OFF state, and a selecting button for mode and settings of the wearable device. Within the scope of the present invention, it is also conceivable with embodiments where the user interface comprises other means for communication with or control of the wearable device, for example a function capable of identifying hand movements by the user close to the first sensor device. Such a function enables the user to control the wearable device and change settings, for example, by placing a finger against the sensor for a certain period of time or with a specific time pattern.

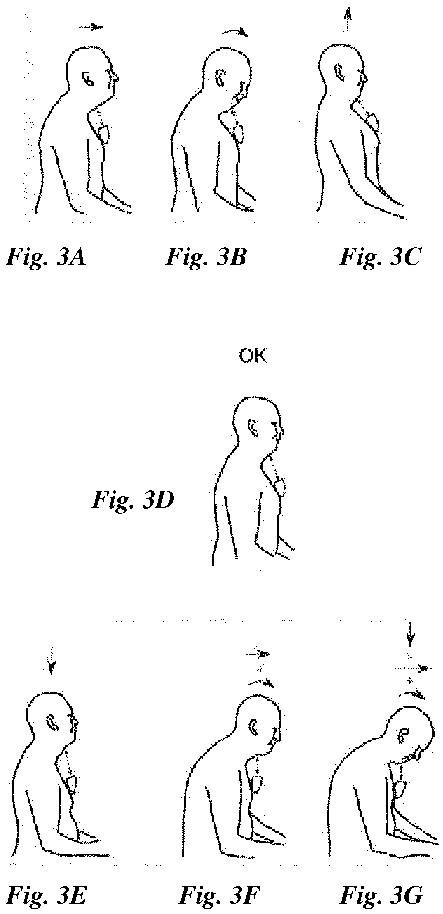

The wearable device for monitoring body posture according to the present invention has many advantages. In addition to its capability of monitoring head, neck and spine posture in order to counteract degradation of posture, as schematically illustrated in Figures 3A-3G, it can also increase the user's awareness of body mechanics and be used as a training device helping the user to obtain better posture.

In the foregoing, the present invention has been described with the aid of a number of different embodiments and with reference to the accompanying drawings. It should be understood, however, that the invention is not limited to the described embodiments and to what is shown in drawings, but that also other embodiments are conceivable within the scope of the invention as it is defined by the following claims.

## Claims

1. A wearable device for monitoring body posture, said wearable device (100) comprising
a housing (101) exhibiting a back surface and a front surface,
a fastening means (102) adapted to secure the housing (101) to a user's body,
a first sensor device (103) and a second sensor device (104) each being mounted at the wearable device and adapted to measure changes in a user's body posture when the wearable device (100) is worn by a user (U),
a processing unit adapted to collect and process sensor signals delivered from said sensor devices (103, 104) and to generate and transmit output signals, and
at least one feedback device adapted to receive said output signals from the processing unit and to provide posture feedback to a user wearing the wearable device,
wherein the processing unit (105) is configured to decide when to transmit a control signal to the feedback device (106) causing it to provide posture feedback to a user, based upon said signals delivered by the sensors, and
wherein the at least one feedback device (106) is configured to provide posture feedback when receiving the control signal from the processing unit (105),
***characterized in***
**that** the fastening means (102) is adapted to secure the housing (101) adjacent to a user's sternum (S) with the back surface facing a user's body when the wearable device is worn,
**that** the first sensor device (103) is mounted at the housing and configured to deliver a signal corresponding to an actual distance (X) between the front surface of the housing (101) and a user's chin (C) when the wearable device is worn,
**that** the second sensor device (104) is mounted at the housing and configured to deliver a signal corresponding to an actual inclination (α) of a user's sternum (S) when the wearable device is worn, and
**that** the processing unit (105) is configured to, when the wearable device is worn by a user, determine both inclination and translation (FT) of a user's head by calculating both head tilt and displacement based on the signals from the second sensor device (104) corresponding to the inclination (a) of the sternum (S), and the signals from the first sensor device (103) corresponding to the distance (X) between the front surface of the housing (101) and the chin (C).

2. The wearable device according to claim 1, **characterized in that** the housing (101) exhibits sloping side surfaces connecting said back surface to said front surface, so that the back surface and the side surfaces together form a wedge-shape adapted to increase friction against a user's clothing.

3. The wearable device according to claim 1 or 2, **characterized in that** the first sensor device is a contactless distance measurement sensor (103) mounted at the front surface of the housing (101).

4. The wearable device according to any one of the preceding claims, **characterized in that** the first sensor device (103) is an infrared sensor or a radar sensor, preferably a time-of-flight sensor.

5. The wearable device according to any one of the preceding claims, **characterized in that** the second sensor device is an inclination measurement sensor (104) mounted in the housing (101).

6. The wearable device according to any one of the preceding claims, **characterized in that** the wearable device (100) further comprises a third sensor device in the form of an accelerometer configured to measure a user's motion activity.

7. The wearable device according to any one of the preceding claims, **characterized in that** the fastening means comprises a carrying strap (102) adapted to be fitted around a user's neck (N).

8. The wearable device according to claim 7, **characterized in that** the carrying strap (102) has the feedback device (106) adjustably mounted thereon to allow the feedback device to be placed in a position that is close to the middle of the back of the user's neck (N) when the wearable device (100) is worn by the user (U).

9. The wearable device according to any one of the preceding claims, **characterized in that** the feedback device (106) comprises a vibrating element intended to be in contact with the user's body when the wearable device (100) is worn by the user (U).

10. The wearable device according to any one of the preceding claims, **characterized in that** the wearable device (100) is provided with an ON/OFF power switch in the form of an electrical connector with mating separable contacts, a mechanical device actuating a switch in the wearable device, or a magnet-actuated switch, which ON/OFF power switch is mounted on or integrated with the fastening means (102).

11. The wearable device according to any one of the preceding claims, **characterized in that** the wearable device (100) is provided with a computer system, which may include said processing unit (105), wherein the computer system further comprises a non-volatile memory for storing data on a user's training development, a radio communication unit for transmitting data to and receiving data from an external unit, and an interface for controlling a user interface of the wearable device (100).

## Patentansprüche

1. Tragbare Vorrichtung zur Überwachung der Körperhaltung, dabei umfasst die tragbare Vorrichtung (100)
ein Gehäuse (101), das eine Rückseite und eine Vorderseite aufweist,
Befestigungsmittel (102) zum Befestigen des Gehäuses (101) am Körper des Benutzers,
eine erste Sensorvorrichtung (103) und eine zweite Sensorvorrichtung (104), die jeweils an das tragbare Gerät montiert und dazu geeignet sind, Änderungen in der Körperhaltung des Benutzers zu messen, wenn das tragbare Gerät (100) vom Benutzer (U) getragen wird,
eine Verarbeitungseinheit zum Sammeln und Verarbeiten von Sensorsignalen, die von Sensoreinrichtungen (103, 104) geliefert werden sowie zum Erzeugen und Übertragen von Ausgangssignalen und
mindestens eine Rückkopplungsvorrichtung, die dazu geeignet ist, die Ausgangssignale von der Verarbeitungseinheit zu empfangen und um dem Benutzer, der die tragbare Vorrichtung trägt, eine Haltungsrückmeldung zu geben,
wobei die Verarbeitungseinheit (105) dazu konfiguriert ist, zu entscheiden, wann ein Signal an die Rückkopplungsvorrichtung (106) gesendet werden soll, welches bewirkt, dass sie dem Benutzer eine Haltungsrückkopplung liefert, die Haltungsrückkopplung basiert auf den von den Sensoren gelieferten Signalen und
das die mindestens eine Rückkopplungsvorrichtung (106) dazu konfiguriert ist, eine Haltungsrückkopplung, beim Empfang des Steuersignals von der Verarbeitungseinheit (105), bereitzustellen welche
**dadurch gekennzeichnet ist,**
**dass** das Befestigungsmittel (102) darauf ausgerichtet ist, das Gehäuse (101) vor dem Sternum (S) des Benutzers zu befestigen, wobei die Rückseite dem Körper des Benutzers zugewandt ist, wenn das Gerät getragen wird,
**dass** die erste Sensoreinrichtung (103) am Gehäuse montiert und zur Aussendung eines Signales konfiguriert ist, welches sich auf den tatsächlichen Abstand (X) zwischen der Oberfläche der Vorderseite des Gehäuses (101) und dem Kinn (C) des Benutzers Sternum bezieht, wenn das tragbare Gerät getragen wird,
**dass** die zweite Sensoreinrichtung (104) am Gehäuse montiert und dazu konfiguriert ist,
ein Signal zu senden, das der tatsächlichen Neigung (α) des Sternum (S) des Benutzers entspricht, wenn das tragbare Gerät getragen wird und
**dass** die Verarbeitungseinheit (105) dazu konfiguriert ist, sowohl die Neigung als auch die Translation (FT) des Kopfes des Benutzers zu bestimmen, welche auf der Berechnung der Kopfneigung und seiner Verschiebung, basierend auf den Signalen der zweiten Sensorvorrichtung (104) welche der Neigung (α) des Sternums (S) und den Signalen der erste Sensoreinrichtung (103) welche dem Abstand (X) zwischen der Oberfläche der Vorderseite des Gehäuses (101) und dem Kinns (C) entsprechen, wenn das tragbare Gerät von einem Benutzer getragen wird .

2. Das tragbare Gerät nach Patentsnspruch 1, ist **dadurch gekennzeichnet, dass** das Gehäuse (101) geneigte Seitenflächen aufweist, die die Rückseite mit der Vorderseite verbinden, so dass die Rückfläche und die Seitenflächen zusammen eine an Keilform bilden angepasst zur Erhöhung der Reibung auf der Kleidung des Benutzers.

3. Das tragbare Gerät nach Patentanspruch 1 oder 2, ist **dadurch gekennzeichnet, dass** die erste Sensoreinrichtung ein berührungsloser Abstandsmesser (103) ist, der an der Oberfläche der Vorderseite des Gehäuses (101) angebracht ist.

4. Das tragbare Gerät nach einem der vorangehenden Patentansprüche, ist **dadurch gekennzeichnet, dass** die erste Sensoreinrichtung (103) ein Infrarotsensor oder ein Radarsensor ist, vorzugsweise ein Laufzeitsensor (time-of-flight-sensor).

5. Das tragbare Gerät nach einem der vorangehenden Parentansprüche, ist **dadurch gekennzeichnet, dass** die zweite Sensoreinrichtung ,ein Neigungsmesssensor (104), im Gehäuse (101) montiert ist.

6. Das tragbare Gerät nach einem der vorangehenden Patentansprüche, ist **dadurch gekennzeichnet, dass** die tragbare Vorrichtung (100) ferner eine dritte Sensorvorrichtung in der Form eines Beschleunigungsmessers umfasst, der zum Messen der Bewegungsaktivität des Benutzers konfiguriert ist.

7. Das tragbare Gerät nach einem der vorangehenden Patentsnsprüche, ist **dadurch gekennzeichnet, dass** das Befestigungsmittel einen anpassbaren Tragegurt (102) umfasst, welcher dazu ausgelegt ist um der Nacken deses Benutzers (N) zu passen.

8. Das tragbare Gerät nach Patentanspruch 7, ist **dadurch gekennzeichnet, dass** die Rückkopplungsvorrichtung (106) verstellbar am Tragegurt (102) angebracht ist, damit das Rückmeldegerät in einer Position nahe der Mitte der Rückseite der Nacken (N) des Benutzers plaziert werden kann, wenn das tragbare Gerät (100) vom Benutzer (U) getragen wird.

9. Das tragbare Gerät nach einem der vorangehenden Patentansprüche, ist **dadurch gekennzeichnet, dass** die Rückkopplungsvorrichtung (106) ein Vibrationselement umfasst, welches sich im Kontakt mit dem Körper des Benutzers befinden soll, wenn das tragbare Gerät (100) vom Benutzer (U) getragen wird.

10. Das tragbare Gerät nach einem der vorangehenden Patentansprüche, ist **dadurch gekennzeichnet, dass** das tragbare Gerät (100) mit einem EIN/AUS-Netzschalter in der Form eines elektrischen Steckverbinders mit zusammenpassenden trennbaren Kontakten, einem mechanischen Gerät welches einen Schalter im tragbaren Gerät betätigt oder eines Magnetkraftschalters, Der EIN/AUS-Netzschalter ist am Befestigungsmittel (102) angebracht oder in dieses integriert.

11. Das tragbare Gerät nach einem der vorangehenden Patentansprüche, ist **dadurch gekennzeichnet, dass** das tragbare Gerät (100) mit einem Computersystem versehen ist, das die Verarbeitungseinheit (105) umfassen kann, wobei das Computersystem ferner Folgendes umfasst: einen nichtflüchtigen Speicher zum Speichern von Daten über die Trainingsentwicklung des Benutzers, eine Funkeinheit zum Senden von Daten und Empfangen von Daten von einer externen Einheit, und eine Schnittstelle zur Steuerung des tragbaren Geräts (100) mit Hilfe einer Benutzeroberfläche.

## Revendications

1. Dispositif portable pour surveiller la posture corporelle, ledit dispositif portable (100) comprenant
un boîtier (101) présentant une surface arrière et une surface avant,
un moyen de fixation (102) adapté pour fixer le boîtier (101) au corps d'un utilisateur,
un premier dispositif capteur (103) et un second dispositif capteur (104) montés chacun au dispositif portable et adapté pour mesurer les changements de la posture du corps d'un utilisateur lorsque le dispositif portable (100) est porté par un utilisateur (U),
une unité de traitement adaptée pour collecter et traiter les signaux des capteurs délivrés par lesdits des capteurs (103, 104) et pour générer et transmettre des signaux de sortie, et
au moins un dispositif de rétroaction adapté pour recevoir lesdits signaux de sortie de l'unité de traitement et pour fournir une rétroaction de posture à un utilisateur portant le portable dispositif,
dans lequel l'unité de traitement (105) est configurée pour décider quand transmettre un signal de commande un signal au dispositif de rétroaction (106) l'amenant à fournir une rétroaction de posture à un utilisateur, sur la base desdits signaux délivrés par les capteurs, et
dans lequel au moins un dispositif de rétroaction (106) est configuré pour fournir une rétroaction de posture lors de la réception du signal de commande de l'unité de traitement (105),
caractérisé en
en ce que le moyen de fixation (102) est adapté pour fixer le boîtier (101) adjacent au sternum (S) d'un utilisateur, la surface arrière orientée face au corps d'un utilisateur lorsque le dispotif portable est porté,
que le premier dispositif capteur (103) est monté sur le boîtier et configuré pour fournir un signal relatif correspondant à une distance réelle (X) entre la surface avant de le boîtier (101) et le menton (C) d'un utilisateur lorsque le dispositif portable est porté,
que le deuxième dispositif capteur (104) est monté sur le boîtier et configuré pour délivrer un signal relatif correspondant à une inclinaison réelle (a) du sternum (S) d'un utilisateur lorsque le dispositif portable est porté, et
que l'unité de traitement (105) est configurée pour, lorsque le dispositif portable est porté par un l'utilisateur, déterminer à la fois l'inclinaison et la translation (FT) de la tête d'un utilisateur en calculant inclinaison et le déplacement de la tête sur la base des signaux du deuxième dispositif capteur (104) correspondant à l'inclinaison (a) du sternum (S), et aux signaux du premier dispositif capteur (103) correspondant à la distance (X) entre la face avant du boîtier (101) et du menton (C).

2. Dispositif portable selon la revendication 1, **caractérisé en ce que** le boîtier (101) présente des surfaces latérales inclinées reliant ladite surface arrière à ladite surface avant, de sorte que la surface arrière et les surfaces latérales forment ensemble une forme de coin adapté à augmenter la friction contre les vêtements d'un utilisateur.

3. Dispositif portable selon la revendication 1 ou 2, **caractérisé en ce que** le premier dispositif capteur est un capteur de mesure de distance sans contact (103) monté sur la surface avant du boîtier (101).

4. Dispositif portable selon l'une quelconque des revendications précédentes, **caractérisé en ce que** le premier dispositif capteur (103) est un capteur infrarouge ou un capteur radar, de preference un capteur de temps de vol (time-of-flight).

5. Dispositif portable selon l'une quelconque des revendications précédentes, **caractérisé en ce que** le deuxième dispositif capteur est un capteur de mesure d'inclinaison (104) monté dans le boîtier (101).

6. Dispositif portable selon l'une quelconque des revendications précédentes, **caractérisé en ce que** le dispositif portable (100) comprend en outre un troisième dispositif capteur sous la forme d'un accéléromètre configuré pour mesurer l'activité de mouvement d'un utilisateur.

7. Dispositif portable selon l'une quelconque des revendications précédentes, **caractérisé en ce que** le moyen de fixation comprend une sangle de transport (102) adaptée pour être ajustée autour du cou d'un utilisateur (N).

8. Dispositif portable selon la revendication 7, **caractérisé en ce que** la sangle de transport (102) a le dispositif de rétroaction (106) monté de manière réglable sur celle-ci pour permeitre au dispositif de rétroaction d'etre placé dans une position proche du milieu de la nuqve l'arrière du de l'utilisateur (N) lorsque le dispositif portable (100) est porté par l'utilisateur (U).

9. Dispositif portable selon l'une quelconque des revendications précédentes, **caractérisé en ce que** le dispositif de rétroaction (106) comprend un élément vibrant destiné à être en contact avec le corps de l'utilisateur lorsque le dispositif portable (100) est porté par l'utilisateur (U).

10. Dispositif portable selon l'une quelconque des revendications précédentes, **caractérisé en ce que** le dispositif portable (100) est pourvu d'un interrupteur marche / arrêt dans le sous la forme d'un connecteur électrique avec des contacts séparables accouplés, un dispositif mécanique actionnant un interrupteur dans le dispositif portable, ou un interrupteur actionné par aimant, sur le quell l'interrupteur marche / arrêt est monté ou dans lequel il est intégré au moyen de fixation (102).

11. Dispositif portable selon l'une quelconque des revendications précédentes, **caractérisé en ce que** le dispositif portable (100) est pourvu d'un système informatique, qui peut compreandre ladite unité de traitement (105), dans lequel le système informatique comprend en outre une mémoire non volatile pour stocker des données sur l'evalution de la l'entrainement d'un utilisateur, une unité de communication radio pour transmettre et recevoir des données d'une unité externe, et une interface pour contrôler une interface utilisateur du dispositif portable (100).
